# EUROPEAN PATENT APPLICATION

(11) **EP 3 101 139 A1**
(43) Date of publication of application: **07.12.2016**
(21) Application number: 14880989.0
(22) Date of filing: 28.01.2014
(51) Int. Cl.: C12P 19/56

(54) **REBAUDIOSIDE M ENZYMATIC PREPARATION METHOD**

(71) Applicant: Pepsico, Inc., Purchase, NY 10577 (US)
(72) Inventor: TAO, Junhua, Zhangjiagang Jiangsu 215600 (CN); LI, Guoqing, Zhangjiagang Jiangsu 215600 (CN); LIANG, Xiaoliang, Zhangjiagang Jiangsu 215600 (CN); LEE, Thomas, Hawthorne, New York 10532 (US); YEP, Gregory, Hawthorne, New York 10532 (US); HOU, Maoqi, Zhangjiagang Jiangsu 215600 (CN); TAO, Andrew, Hawthorne, New York 10532 (US)
(74) Representative: Jenkins, Peter David
(86) International application number: PCT/CN2014/071715
(87) International publication number: WO 2015/113231

(57) **Abstract**

Provided is a method for preparing Rebaudioside M by using an enzyme method. In the method, Rebaudioside A or Rebaudioside D is used as a substrate, and in the presence of sucrose and UDP, Rebaudioside M is generated by means of reaction of the substrate under the catalysis of a mixture of UDP-glucosyl transferase and sucrose synthetase, or recombinant cells containing the UDP-glucosyl transferase and sucrose synthetase. The reaction is carried out in an aqueous-phase system at a temperature of 20 to 60°C and pH 5.0 to 9.0. The reaction system further contains dimethyl sulfoxide at a concentration of 3% to 5% according to the ratio by volume for facilitating solubilization of the substrate.

## Description

### Technical Field

The present invention relates to a method for preparing Rebaudioside M, and in particular to a biological method for preparing Rebaudioside M.

### Background

Sugar substitutes can be divided into 4 classes with different influences on human health. The first class includes natural sweeteners such as fruit juices, honey and maple syrup, which are similar to sugar in terms of calorie and carbohydrate content. The second class includes artificial sweeteners, such as aspartame and saccharin that provide no calories and are thus considered as non-nutritive sweeteners. The third class includes sugar alcohols, such as xylitol and sorbitol, mainly derived from vegetables and fruits. The fourth class includes sweet substances extracted from natural plants, such as stevioside extracted from *Stevia rebaudiana.* Artificial sweeteners, sugar alcohol and extracted sweeteners all taste many times - some hundreds of times - sweeter than sugar, so that the amounts added needed to create the same level of sweetness are dramatically lower than sucrose.

*Stevia rebaudiana* is a crop of important economic value, and has a very high content of stevioside in the leaves thereof. At present, more than 100 compounds have been identified in *Stevia rebaudiana,* and the best known one is stevioside, and in particular, steviol glycoside and Rebaudioside A (J. Agric. Food Chem., 2012, 60, 886-895). Recently a novel stevioside has been found in a hybrid *Stevia rebaudiana* plant Morita (2010, J. Appl. Glycosci., 57, 199-209).

Patent Application No. 201310353500.9 discloses a biological method for preparing Rebaudioside M, where Rebaudioside A or Rebaudioside D is used as a substrate, and in the presence of a glucosyl donor, Rebaudioside M is generated by means of reaction of the substrate under the catalysis of UDP-glucosyl transferase and/or recombinant cells containing the UDP-glucosyl transferase. However, the process of the patent is not sufficiently optimized, with a conversion rate of only about 80% at most and a purity of only 95%, as well as a lower substrate concentration and a higher production cost. Thus, the process is not suitable for industrialized production.

### Summary

A technical issue to be solved in the present invention is to provide a method for preparing Rebaudioside M by an enzyme method in order to overcome drawbacks in the prior art. This method can produce a Rebaudioside M product with a higher purity at a lower cost.

In order to solve the above technical issue, the present invention employs the following technical solution:
A method for preparing Rebaudioside M by using an enzyme method, where Rebaudioside A or Rebaudioside D is used as a substrate, and in the presence of sucrose and UDP, Rebaudioside M is generated by means of reaction of the substrate under the catalysis of a mixture of UDP-glucosyl transferase and sucrose synthetase (AtSUS1), or recombinant cells containing the UDP-glucosyl transferase and sucrose synthetase. The reaction is carried out in an aqueous-phase system at a temperature of 20 to 60°C and pH 5.0 to 9.0. The reaction system further contains dimethyl sulfoxide at a concentration of 3% to 5% according to the ratio by volume for facilitating solubilization of the substrate. In the initial reaction system, the Rebaudioside A has a concentration greater than or equal to 10 g/L, and the Rebaudioside D has a concentration greater than or equal to 15 g/L. After the reaction, the reaction solution is centrifuged, then the supernatant is taken, and separated with a macroporous adsorbent resin to obtain a solution of a crude product of Rebaudioside M, which is crystallized in an aqueous ethanol solution, so as to obtain a Rebaudioside M product with a purity greater than 98%.

Preferably, the UDP-glucosyl transferase is UGT-A derived from *Stevia rebaudiana* and / or UGT-B derived from *Oryza sativa.*

Preferably, the reaction is carried out in an aqueous-phase system at a temperature of 35°C to 45°C and pH 6.5 to 8.5.

Preferably, the recombinant cells containing UDP-glucosyl transferase and sucrose synthetase are employed to carry out the catalysis, and the initial reaction system further contains toluene at a concentration of 1% to 3% according to the ratio by volume.

Preferably, a ratio by weight of the UDP-glucosyl transferase to the sucrose synthetase is 1:0.2 to 0.4.

Preferably, the method is implemented as follows: all the raw materials employed in the reaction are added into a reaction kettle, brought to a final volume with an aqueous-phase system, mixed uniformly, then placed at a set temperature, and stirred for reaction.

Preferably, the recombinant cells are microbial cells.

More preferably, the microorganism is *Escherichia coli, Saccharomyces cerevisiae* or *Pichia pastoris.*

Preferably, the substrate is Rebaudioside A, and the UDP-glucosyl transferase is a mixture of UGT-A derived from *Stevia rebaudiana* and UGT-B derived from *Oryza sativa,* where the amino acid sequence of UGT-A derived from *Stevia rebaudiana* is at least 80% consistent with Sequence 2, and the amino acid sequence of UGT-B derived from *Oryza sativa* is at least 80% consistent with Sequence 4. More preferably, in the mixture of UGT-A derived from *Stevia rebaudiana* and UGT-B derived from *Oryza sativa,* a ratio by weight of UGT-A derived from *Stevia rebaudiana* to UGT-B derived from *Oryza sativa* is 3 to 5:1.

Preferably, the substrate is Rebaudioside D, the UDP-glucosyl transferase is UGT-A derived from *Stevia rebaudiana,* and the amino acid sequence of UGT-A derived from *Stevia rebaudiana* is at least 80% consistent with Sequence 2.

Preferably, the reaction solution, after the reaction and before the centrifugation, is first heated at 50 to 60°C and subjected to ultrasonic treatment for 50 to 70 min.

Preferably, the solution of the crude product of Rebaudioside M is crystallized in the aqueous ethanol solution by particular steps as follows: the solution of the crude product of Rebaudioside M is concentrated by distillation at a reduced pressure and then centrifuged, and the supernatant is discarded; the precipitate is washed with added water and then centrifuged, and the supernatant is discarded; the precipitate is suspended with an aqueous ethanol solution at a concentration of 40% to 70% according to the ratio by volume, heated to 60-70°C for solubilization, and water is added therein until the aqueous ethanol solution has a concentration of 20 to 30% according to the ratio by volume; and the mixture is gradually cooled to room temperature, and subjected to crystallization and precipitation of a solid, followed by suction filtration and vacuum drying.

As a result of implementation of the above technical solutions, the present invention possesses the following advantages as compared with the prior art.

The method for preparing Rebaudioside M by an enzyme method provided in the present invention has an important application value. Through optimization and controlling of the solubilizer, reaction temperature and pH, the conversion rate and purity are both improved, and the production cost is reduced. Thus, this method is suitable for industrialized production.

### Brief Description of the Drawings

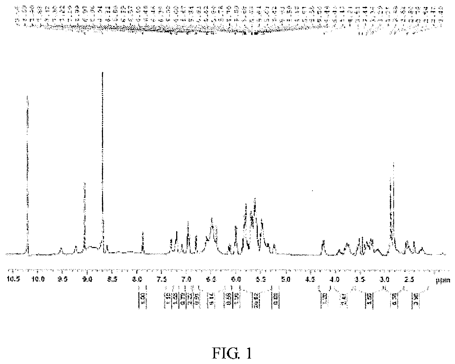

FIG. 1 is a proton magnetic spectrum diagram of a product obtained in Example 7 of the present invention.

### Detailed Description of the Preferred Embodiments

The present invention provides a process for synthesizing Rebaudioside M by using an enzyme method, with Rebaudioside A or Rebaudioside D as a raw material.

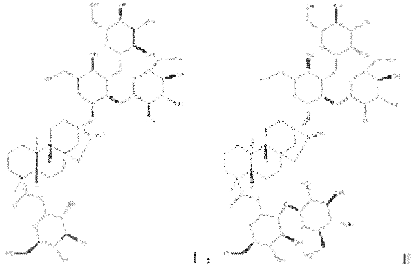

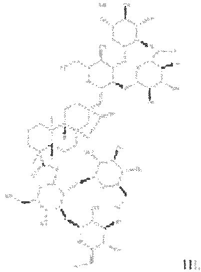

Rebaudioside A, Rebaudioside D and Rebaudioside M contain 4, 5 and 6 dextrose units of stevioside respectively, with structural formulae respectively seen in Formulae I, II and III.

The present invention provides two routes for synthesizing Rebaudioside M:
Route 1: 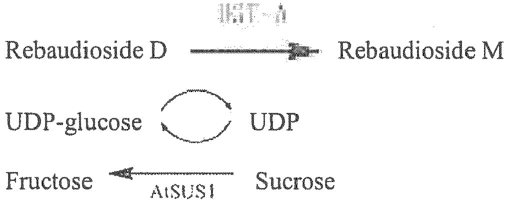
Route 2:

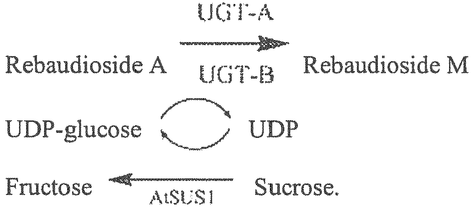

In the present invention, the raw material is purified.

According to one particular aspect of the present invention, the raw material comprises a steviol glycoside substrate Rebaudioside A.

According to another particular aspect of the present invention, the raw material comprises a steviol glycoside substrate Rebaudioside D.

According to the present invention, the UDP glucosyl transferase may be occurred in a form of a lyophilized enzyme powder that may or may not have been purified, or present in recombinant cells.

Recombinant cells containing UGT-A, UGT-B and AtSUS1 (a sucrose synthetase) are obtained by the following method.

Recombinant *Escherichia coli* (or other microbial bacteria) expression strains of UGT-A, UGT-B and AtSUS1 are obtained by utilizing molecular cloning techniques and genetic engineering techniques. Then, the recombinant *Escherichia coli* are fermented, and subjected to aftertreatment and collection of the recombinant cells.

The molecular cloning techniques and genetic engineering techniques described in the present invention are all known. Molecular cloning techniques may be seen in Molecular Cloning: A Laboratory Manual. 3rd Edition, by J. Shambrook, 2005.

Expression steps for constructing the recombinant strains of the present invention by employing the genetic engineering technique are as follows:
(1) gene fragments of UGT-A and AtSUS1 are subcloned respectively into two multiclonal sites MCS2 (NdeI/XhoI) and MCS1 (BamHI/HindIII) of pACYC-Duet-1, to obtain a plasmid pA-UGTA-SUS1;
(2) the UGT-B gene is subcloned into a site between the NdeI and BamHI sites of pET30a, to obtain a recombinant plasmid pE-UGIT-B; and
(3) PA-UGT-A-SLTS1 and pE-UGT-B are transformed successively into *Escherichia coli* BL21 (DE3), to obtain a strain GQ-ABS.

The recombinant cells containing UGT, or the lyophilized UGT powders are prepared by utilizing the recombinant *Escherichia coli* expression strains containing UGT, by steps as follows:
The recombinant *Escherichia coli* expression strain GQ-ABS is inoculated into 4 mL of a liquid LB medium according to a proportion of 1%, and cultured overnight with shaking (200 rpm) at 37°C. The culture that has experienced overnight culturing is transferred to 50 mL of the liquid LB medium in an inoculum size of 1%. The culture medium is cultured with shaking (200 rpm) at 37°C to an OD600 value up to 0.6-0.8. IPTG at a final concentration of 0.4 mM is added therein, and the mixture is cultured overnight with shaking at 20°C. After completion of the induction, cells are collected by centrifugation (8,000 rpm, 10 min). The cells are resuspended using 5 mL of a 2 mmol/L phosphate buffer (pH 7.0) to obtain the recombinant cells, or further ruptured ultrasonically in an ice bath, to obtain the lyophilized powder by centrifuging the ruptured liquid (8,000 rpm, 10 min), collecting the supernatant and lyophilizing for 24 h.

The present invention will be described below in more details in conjunction with particular examples.

### Example 1: preparation of recombinant Escherichia coli cells containing UGT-A

According to Sequence 1 and Sequence 2, UGT-A gene fragments were genetically synthesized, NdeI and BamHI enzyme cutting sites were added on both ends respectively, and pUC57 vectors (Suzhou Genewiz Biotech Co., Ltd.) were ligated therein. The UGT gene fragments were subjected to enzyme digestion with restriction endonucleases NdeI and BamHI. Purified fragments were recovered. T4 ligase was added therein, and the fragments were ligated into corresponding enzyme cutting sites of pET30a, to transform the BL21 (DE3) strains, so as to obtain a recombinant strain GQ-A.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-A for use in the catalysis.

### Example 2: preparation of lyophilized UGT-A powder

The recombinant cells of UGT-A prepared in Example 1 were ruptured ultrasonically in an ice bath, to obtain a lyophilized powder of UGT-A by centrifuging the ruptured liquid (8,000 rpm, 10 min), collecting the supernatant and lyophilizing for 24 h.

### Example 3: preparation of recombinant Escherichia coli cells containing UGT-B

According to Sequence 3 and Sequence 4, UGT-B gene fragments were genetically synthesized, NdeI and BamHI enzyme cutting sites were added on both ends respectively, and pUC57 vectors (Suzhou Genewiz Biotech Co., Ltd.) were ligated therein. The UGT gene fragments were subjected to enzyme digestion with restriction endonucleases NdeI and BamHI. Purified fragments were recovered. T4 ligase was added therein, and the fragments were ligated into corresponding enzyme cutting sites of pET30a, to transform the BL21 (DE3) strains, so as to obtain a recombinant strain GQ-B.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-B for use in the catalysis.

### Example 4: preparation of lyophilized UGT-B powder

The recombinant cells of UGT-B prepared in Example 3 were ruptured ultrasonically in an ice bath, to obtain a lyophilized powder of UGT-B by centrifuging the ruptured liquid (8,000 rpm, 10 min), collecting the supernatant and lyophilizing for 24 h.

### Example 5: preparation of recombinant Escherichia coli cells containing UGT-A and AtSUS1

Gene fragments of UGT-A and AtSUS1 were inserted respectively into NdeI/XhoI and BamHI/HindIII sites of the pACYC-Duet-1 plasmid, to obtain a plasmid pA-UGT-A-SUS1. The plasmid was transformed into BL21 (DE3), to obtain a recombinant strain GQ-AS.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-A and AtSUS1 for use in the catalysis.

### Example 6: preparation of recombinant Escherichia coli cells containing UGT-A, UGT-B and AtSUS1

According to Sequence 3 and Sequence 4, UGT-B gene fragments were genetically synthesized, NdeI and BamHI enzyme cutting sites were added on both ends respectively, and pUC57 vectors (Suzhou Genewiz Biotech Co., Ltd.) were ligated therein. The UGT gene fragments were subjected to enzyme digestion with restriction endonucleases NdeI and BamHI. Purified fragments were recovered. T4 ligase was added therein, and the fragments were ligated into corresponding enzyme cutting sites of pET30a, to transform the GQ-AS strains, so as to obtain a recombinant strain GQ-ABS.

The UGT strains were inoculated into 4 mL of a liquid LB medium according to a proportion of 1%, and cultured overnight with shaking (200 rpm) at 37°C. The culture that had experienced overnight culturing was transferred to 50 mL of the liquid LB medium in an inoculum size of 1%. The culture medium was cultured with shaking (200 rpm) at 37°C to an OD600 value up to 0.6-0.8. IPTG at a final concentration of 0.4 mM was added therein, and the mixture was cultured overnight with shaking at 20°C. After completion of the induction, cells were collected by centrifugation (8,000 rpm, 10 min). The cells were resuspended using 5 mL of a 2 mmol/L phosphate buffer (pH 7.0) to obtain recombinant cells containing UGT-A, UGT-B and AtSUS1 for use in the catalysis.

### Example 7: synthesis of Rebaudioside M by an enzyme method with Rebaudioside D as a substrate

0.224 g of UDP, 34.2 g of sucrose, 1.6 g of Rebaudioside D, 1 g of lyophilized UGT-A powder, 0.4 g of lyophilized AtSUS1 powder, 4 mL of dimethyl sulfoxide and 0.05 mol/L phosphate buffer (pH 8.0) were added successively into the reaction system to a final volume of 100 mL, mixed uniformly, then placed in a water bath at 37°C, and stirred at 200 rpm to carry out reaction for 18 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside D was more than 90%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 1.12 g of Rebaudioside M, with a purity greater than 99%.

### Example 8: synthesis of Rebaudioside M by an enzyme method with Rebaudioside A as a substrate

0.18 g of UDP, 41.04 g of sucrose, 1 g of Rebaudioside A, 2 g of lyophilized UGT-A powder, 0.5 g of lyophilized UGT-B powder, 0.5 g of lyophilized AtSUS1 powder, 4 mL of dimethyl sulfoxide and 0.05 mol/L phosphate buffer (pH 7.0) were added successively into the reaction system to a final volume of 100 mL, mixed uniformly, then placed in a water bath at 37°C, and stirred at 200 rpm to carry out reaction for 18 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside A was more than 90%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 0.69 g of Rebaudioside M, with a purity greater than 99%.

### Example 9: synthesis of Rebaudioside M by whole cell synthesis with Rebaudioside D as a substrate

The GQ-AS recombinant cell employed in the example was a recombinant strain containing both UGT-A and AtSUS1.

0.045 g of UDP, 10.26 g of sucrose, 2 mL of toluene, 0.2 g of Rebaudioside D, 10 g of recombinant cells containing GQ-AS, 4 mL of dimethyl sulfoxide and 0.05 mol/L phosphate buffer (pH 8.0) were added successively into the reaction system to a final volume of 100 mL, mixed uniformly, then placed in a water bath at 37°C, and stirred at 200 rpm to carry out reaction for 7 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside D was more than 90%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 0.14 g of Rebaudioside M, with a purity greater than 99%.

### Example 10: synthesis of Rebaudioside M by whole cell synthesis with Rebaudioside A as a substrate

The GQ-ABS recombinant cell employed in the example was a recombinant strain containing UGT-A, UGT-B and AtSUS1 at the same time.

0.045 g of UDP, 10.26 g of sucrose, 2 mL of toluene, 0.2 g of Rebaudioside A, 10 g of recombinant cells also containing GQ-ABS, 4 mL of dimethyl sulfoxide and 0.05 mol/L phosphate buffer (pH 7.0) were added successively into the reaction system to a final volume of 100 mL, mixed uniformly, then placed in a water bath at 37°C, and stirred at 200 rpm to carry out reaction for 7 h. After completion of the reaction, 200 µl of the reaction solution was taken and added into 800 µl of anhydrous methanol and mixed uniformly. The mixture was centrifuged for 5 min at 10,000 rpm. The supernatant was taken and passed through a filter membrane, followed by detection using high performance liquid chromatography (chromatographic condition: chromatographic column: Agilent eclipse sb-C18 4.6 X 150 mm; detection wavelength: 210 nm; mobile phase: methanol: water = 68%: 32%; flow rate: 1.0 mL/min; column temperature: 30°C). A conversion rate of Rebaudioside A was more than 40%. After completion of the reaction, 300 mL of deionized water was added into 100 mL of the reaction solution, and the mixture was heated for 1 h at 55°C, subjected to ultrasonic treatment, and centrifuged for 30 min at 6,700 rpm. The supernatant was a sample A. After the centrifugation, 100 mL of water was added into the precipitate, and the mixture was heated for 0.5 h at 55°C, subjected to ultrasonic treatment, and centrifuged for 30 min at 6,700 rpm. The supernatant was a sample B. The sample A and the sample B were mixed to then obtain a sample C, which was separated with a macroporous adsorbent resin (AB-8). The resultant was first flushed with 4 column volumes of water, and then eluted with 3.5 column volumes of 70% ethanol, to obtain a solution of the crude product of Rebaudioside M. The above solution of the crude product was distilled at a reduced pressure (40 to 50°C) until the remaining solution was about 10 mL, and centrifuged for 10 min at 9,900 rpm, and the supernatant was discarded. The precipitate was washed with 4 mL of water added, centrifuged for 10 min at 9,900 rpm, and the supernatant was discarded. The precipitate was suspended with a 50% aqueous ethanol solution, and heated to 65°C for solubilization. An equal volume of water was added therein, to an ethanol concentration of 25%. The mixture was gradually cooled to room temperature, and subjected to suction filtration and vacuum drying after precipitation of a solid, to obtain 0.05 g of Rebaudioside M, with a purity greater than 99%.

The above examples are only intended for the description of technical conception and feature of the present invention, for the purpose of enabling those familiar with the art to understand and thereby implement the contents of the present invention, rather than limiting the protection scope of the present invention therewith. Any equivalent changes or modifications made according to the spirit and essence of the present invention shall be encompassed within the protection scope of the present invention.

## Claims

1. A method for preparing Rebaudioside M by using an enzyme method, wherein Rebaudioside A or Rebaudioside D is used as a substrate, and in the presence of sucrose and UDP, Rebaudioside M is generated by means of reaction of the substrate under the catalysis of a mixture of UDP-glucosyl transferase and sucrose synthetase, or recombinant cells containing the UDP-glucosyl transferase and sucrose synthetase, the reaction is carried out in an aqueous-phase system at a temperature of 20 to 60°C and pH 5.0 to 9.0, wherein, in the initial reaction system, the Rebaudioside A has a concentration greater than or equal to 10 g/L, the Rebaudioside D has a concentration greater than or equal to 15 g/L, and after the reaction, the reaction solution is centrifuged, then the supernatant is separated with a macroporous adsorbent resin to obtain a solution of a crude product of Rebaudioside M, which is crystallized in an aqueous ethanol solution, so as to obtain the Rebaudioside M product with a purity greater than 98%.

2. The method according to Claim 1, wherein, in the initial reaction system, the Rebaudioside A has a concentration of 10 to 30 g/L, and the Rebaudioside D has a concentration of 15 to 50 g/L.

3. The method according to Claim 1, wherein, the UDP-glucosyl transferase is UGT-A derived from *Stevia rebaudiana* and/or UGT-B derived from *Oryza sativa.*

4. The method according to Claim 1, wherein, the reaction is carried out in an aqueous-phase system at a temperature of 35°C to 45°C and pH 6.5 to 8.5.

5. The method according to Claim 1, wherein, the recombinant cells containing the UDP-glucosyl transferase and sucrose synthetase are employed in the reaction to carry out the catalysis, and the initial reaction system further contains toluene or other cellular permeating agents at a concentration 1% to 3% according to the ratio by volume.

6. The method according to Claim 1, wherein, a ratio by weight of the UDP-glucosyl transferase to the sucrose synthetase is 1:0.2 to 0. 4.

7. The method according to Claim 1, wherein, the method is implemented as follows: all the raw materials employed in the reaction are added into a reaction kettle, brought to a final volume with an aqueous-phase system, mixed uniformly, then placed at a set temperature, and stirred for reaction.

8. The method according to Claim 1, wherein, the recombinant cells are microbial cells.

9. The method according to Claim 8, wherein, the microorganism is *Escherichia coli, Saccharomyces cerevisiae* or *Pichia pastoris.*

10. The method according to any one of Claims 1 to 9, wherein, the substrate is Rebaudioside A, the UDP-glucosyl transferase is a mixture of UGT-A derived from *Stevia rebaudiana* and UGT-B derived from *Oryza sativa,* wherein the amino acid sequence of UGT-A derived from *Stevia rebaudiana* is at least 80% consistent with Sequence 2, and the amino acid sequence of UGT-B derived from *Oryza sativa* is at least 80% consistent with Sequence 4.

11. The method according to Claim 10, wherein, in the mixture of UGT-A derived from *Stevia rebaudiana* and UGT-B derived from *Oryza sativa,* a ratio by weight of UGT-A derived from *Stevia rebaudiana* to UGT-B derived from *Oryza sativa* is 3 to 5:1.

12. The method according to any one of Claims 1 to 9, wherein, the substrate is Rebaudioside D, the UDP-glucosyl transferase is UGT-A derived from *Stevia rebaudiana,* and the amino acid sequence of UGT-A derived from *Stevia rebaudiana* is at least 80% consistent with Sequence 2.

13. The method according to Claim 1, wherein, the reaction solution, after the reaction and before the centrifugation, is first heated at 50 to 60°C and subjected to ultrasonic treatment for 50 to 70 min.

14. The method according to Claim 1, wherein, the solution of the crude product of Rebaudioside M is crystallized in the aqueous ethanol solution by the particular steps as follows: the solution of the crude product of Rebaudioside M is concentrated by distillation at a reduced pressure and then centrifuged, and the supernatant is discarded; the precipitate is washed with added water and then centrifuged, and the supernatant is discarded; the precipitate is suspended with an aqueous ethanol solution at a concentration of 40% to 70% according to the ratio by volume, heated to 60-70°C for solubilization, and water is added therein until the aqueous ethanol solution has a concentration of 20 to 30% according to the ratio by volume; and the mixture is gradually cooled to room temperature, and subjected to crystallization and precipitation of a solid, followed by suction filtration and vacuum drying.
